# EUROPEAN PATENT SPECIFICATION

(11) **EP 1 449 502 B1**
(45) Date of publication and mention of the grant of the patent: **12.12.2007**
(21) Application number: 03425104.1
(22) Date of filing: 21.02.2003
(51) Int. Cl.: A61F 7/00

(54) **Apparatus for rising body temperature of a person in hypothermic status**
Gerät zur Anhebung der Körperkerntemperatur einer Person im hypothermischen Zustand
Outil pour élever la température du corps d'une personne en état d'hypothérmie

(43) Date of publication of application: 25.08.2004
(73) Proprietor: Flow Meter S.p.a., 24040 Levate (Bergamo) (IT)
(72) Inventor: Paratico, Roberto c/o Flow-Meter S.p.A., 24040 Levate (Bergamo) (IT); Travan, Maurizio, 41037 Mirandola (Modena) (IT)
(74) Representative: Botti, Mario

(56) References cited:
- WO-A-94/04211
- GB-A- 2 277 689
- US-A- 4 601 287
- US-A- 4 951 659
- US-A- 6 068 609

## Description

### Field of Application

The present invention relates to an apparatus for rising body temperature of a person in hypothermia status, e.g. post surgical operation hypotermie.

### Prior Art

In the hospital field it is well-known the problem of surgical operations performed when the patient is in hypothermia status and it essentially deals with the need of restoring the patient's body temperature to normal values.

It is known in fact that during surgical operations that may last several hours or that may be highly invasive, such as organ transplanting and open-heart operations, the body temperature of the patient must be lowered a lot the patient's body temperature and operate in hypothermia conditions. Often, the patient's hypothermie must be maintained constant throughout the operation.

Thus, upon completion of the surgical operation, the body temperature of the patient must be restored to its normal level within the shortest time, yet gradually so that the patient does not have post-operative problems.

There have been prior approaches to meet these requirements.

For instance, to cover the patient's body with heating sheets so as to reach a predetermined, desired body temperature is a well known technique.

This prior approach, although widely used, is not fully satisfactory because of the relatively long time required to bring the patient's body temperature back to the desired value.

Another approach provides the patient's bed to be heated, or tubolar mattresses through which heated air is blown .

The latter method is expensive and inconvenient, and is not substantially faster than the former one.

A major drawback of both these prior methods is the lack of any reintegration of the liquids the patient has lost during the surgical operation, even only for evaporation's heating. Liquid reintegration are provided with other methods, e.g. intravenously, this without being coordinated with the stage of increasing the body temperature of the patient back to normal.

Invasive procedures are also occasionally used, e.g. when the body temperature of the patient has been lowered to an unsafe level, even close to 29°C.

With such procedures, the patient's blood circulatory system is bypassed by making the blood to flow through an extrabodily heating apparatus. Clearly, such procedures are of a highly invasive nature, and could cause risks of contamination for the patient's blood.

A known prior art solution is disclosed in the international patent application WO 94/04211 wherein a nebulizer apparatus for high-humidity oxygen therapy is disclosed. The apparatus comprises a fluid tank, a nebulizer cap, a fluid delivering line connected to the tank, heating means for heating the fluid, and an antibacterial filter connected in the flow path of said fluid. In particular, in document D 1 it is disclosed that said heating means shapes in an electrical resistance heater which is inserted in an inlet of the cap of the nebulizer apparatus.

The underlying technical problem of this invention is to provide a novel apparatus for increasing the body temperature of a person in hypothermia status, having relevant, uniquely simple, non-invasive, cost-efficient and effective structural and functional features so as to overcome the aforementioned drawbacks of the prior art.

### Summary of the Invention

The resolvent idea on which the present invention stands is the one of exploiting the patient's respiratory apparatus and breathing for properly gradual heating air or a filtered air/oxygen mixture at controlled temperature and humidity.

As such, the huge thermal exchange area offered by the bronchial and lung alveoli is here used to achieve restoration of normal temperature in a non-invasive manner and a much shorter time.

On the basis of the above idea, the technical problem is solved by an apparatus as previously indicated and as defined in the characterizing part of Claim 1.

The features and advantages of the method and the apparatus according to the invention should become apparent from the following description of an embodiment thereof, given by way of an indicative and non-limitative example with reference to the accompanying drawings.

### Brief Description of the Drawings

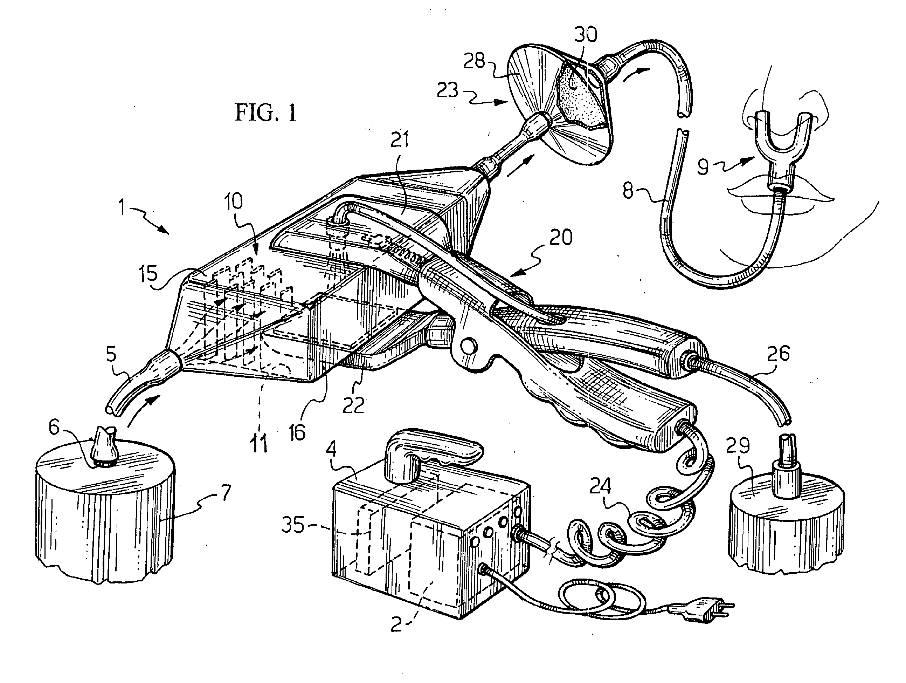

Figure 1 is a schematic perspective view of an apparatus according to the invention.

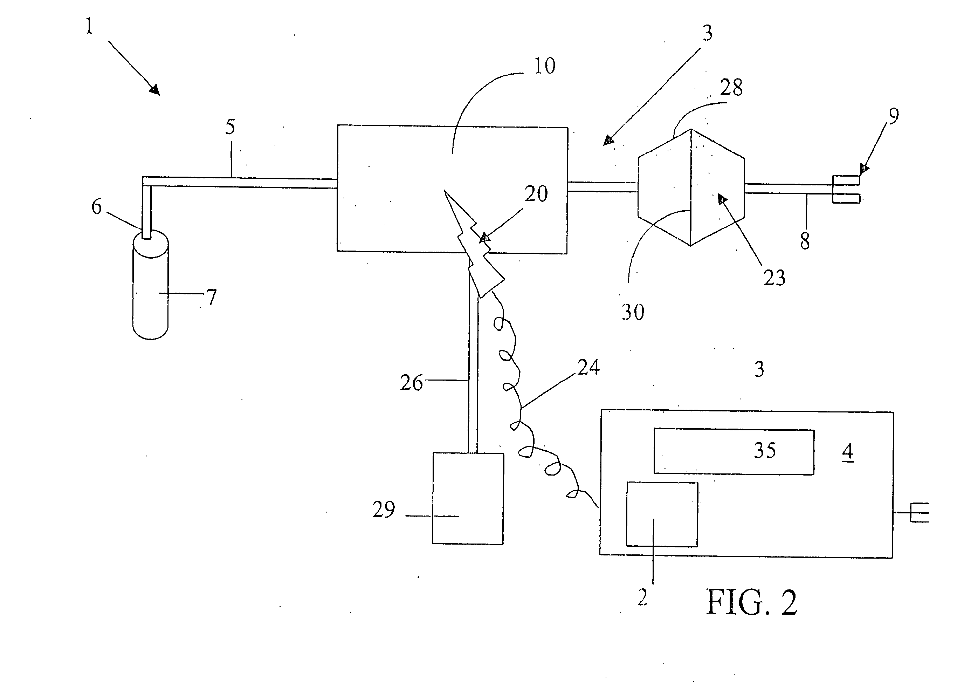

Figure 2 is a schematic block diagram illustrating the operation of the apparatus according to the invention.

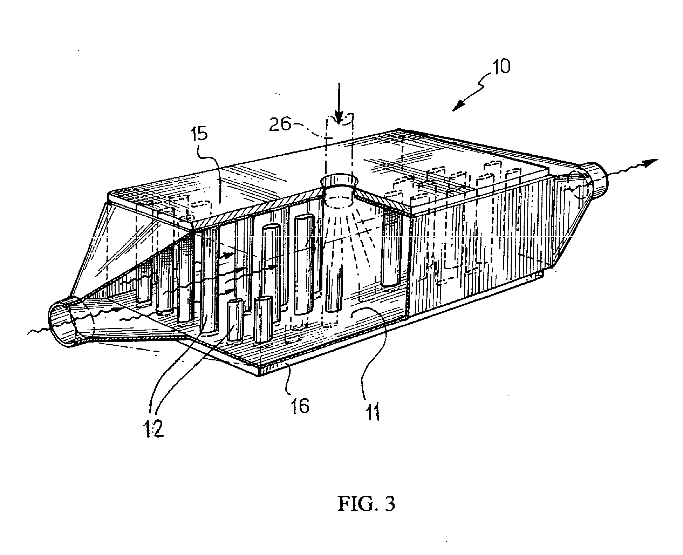

Figure 3 is a schematic partially cutaway view of a detail of the apparatus shown in Figure 1.

### Detailed Description

With reference to the drawings, there is shown an apparatus, generally and in schematic form indicated with 11, according to the invention, for increasing the body temperature of a patient in hypothermia status, e.g. for restoring within the shortest time the body temperature of a patient, directly after an surgical operation.

The apparatus 1 could also be advantageously used during a surgical operation, and whenever a certain patient's body temperature is to be maintained.

Furthermore, the invention would be useful when the body temperature of a patient has to be increased independently from the fact that the patient has been operated or is under anaesthesia, e.g. in case of snow avalanches victims .

The apparatus 1 is a portable and electrically supplied by a battery 2.

The apparatus 1 comprises a portion 3 to be associated with the patient and a removable unity 4 to supervise and control.

The portion 3, to be associated to the patient, comprises a heat exchanger 10 connected at one end to a medical gas tank 7 through a delivering line 5 having an end 6 associated with the tank 7, and at the other end is connected to nasal inhalation end 9 or, alternatively, to a mask for the patient's breathing.

The tank 7 may contain a pressurized gas fluid, such as air, air/oxygen mixture, anaesthetic gas, or any other medical gas mixture to be administered to the patient, with controlled valve regulation.

Advantageously, a heat exchanger 10 and a hydrophobic membrane filter 23, to be described below, are inserted on the line 5. According to a preferred embodiment, said heat exchanger 10 includes a pair of plates 15, 16 which are set apart from each other and stiffened by a set of foils 12 which can define winding or labyrinth-likc flowpaths for the fluid passing through the exchanger. This heat exchanger acts essentially as a radiator.

In the heat exchanger 10 a chamber11 is thus formed, which is meant to be crossed by said fluid. Advantageously, the chamber 11 accommodates the above-referred set of foils 12.

The filter 23 is located downstream the heat exchanger 10, towards the patient.

Outside the heat exchanger 10 looks like a flattened parallelepipedic body provided with two oppositely-located flat surfaces, equipped with metal plates 15, 16, as if they were two capacitor plates.

The metal plates 15, 16 are connected to the foils 12of the chamber 11.

A hole is also provided, passing through one of the surfaces and in communication with the chamber 12. As described hereinafter, demineralized water will be supplied into the chamber of the heat exchanger 10 through this hole.

The body of the heat exchanger 10 is made of a thermoplastic material and is disposable. That is, the heat exchanger 10 is removably arranged on the line 5 and to be discarded after use. Alternatively, the line 5 could be integral with the heat exchanger and in turn discarded together with the latter.

A heating nipper 20 is associated with the unit 4 of the apparatus 1, the nipper having two jaws 21 and 22 arranged to clamp the body tightly in contact with the metal plates 15, 16 of the heat exchanger 10. The nipper 20 removably grasps the body of the heat exchanger 10 for a sufficient length of time to transfer a predetermined amount of heat to the metal plates, and thus to the heat exchange foils 12 in the chamber 11.

In a preferred embodiment, the nipper 20 is formed with conductive material whose resistivity is prefixed so as to allow both jaws 21, 22 to be quickly heated by current flowing through them, or by Peltier-effect cells with thermostat function. The nipper 20 is electrically connected to the control unit 4 of the apparatus 1 by properly protected, flexible cables 24.

The nipper 20 further includes a thermal probe, e.g. an NTC probe or a thermocouple.

The thermal probe is electrically connected to the control unit 4 of the apparatus 1.

Advantageously, a demineralized water supply line 26 is also arranged for feeding the chamber 11 of the heat exchanger 10.

The line 26 comprises an extended section that is held parallel to the nipper 20 and is led through a handle of the latter to anend of one 21 of the jaws. The line 26 terminates with a nozzle, slightly projecting out of the jaw 21, the nozzle being adapted for engagement in the hole passing through the plate 16 and surface 14, and for reaching the chamber 1 of the heat exchanger 10. The heating effect of the gas flow combines in the chamber 11 with a humidifying effect of the same flow because of water being flown across the thermal transfer, heated surfaces.

The inlet end of the line 26, opposite to the nozzle, is associated with a tank 29 of demineralized water.

The filter 23 placed downstream the heat exchanger 10 comprises a housing 28, including a couple of frustoconical half-shells of a thermoplastic material being meant to centrally hold a hydrophobic membrane 30 made of a water-proof but not-vapour-proof material.

The membrane 30 also performs an antibacterial function.

This membrane may be as disclosed in European Patent No. 0 659 096 to the Applicant.

Also the filter 23 can be of the disposable type. In a modified embodiment, the hydrophobic and/or antibacterial filter can be incorporated into the heat exchanger 10.

The combined action of the heat exchanger 10 and the membrane filter 23 allows the gas flow to deliver to the patient, to be heated and humified.

The content of relative humidity in the gas flow should be 100% at the heating temperature. However, as membrane 30 acts as a barrier and allows only vapor to pass directly therethrough, no liquid water can reach the patient. The membrane 30 also holds back condensation water due to the patient's breathing out.

The control unit 4 of the apparatus 1 includes, as mentioned, a power supply and storage battery 2, which can be connected to the electric network for charging or buffering purposes.

The unit 4 further comprises a control logic structure 35 arranged to receive signals from the thermal probe of the nipper 20, and it drives the power supply of the nipper 20 itself in order to regulate the temperature of the jaws 21, 22 and, as a consequence, through thermal conduction, also the interior temperature of the heat exchanger 10.

The medical device of the invention is particularly simple and effective, and dog brilliantly solve the technical problem of bringing subjects and/or patients in hypothermia status back to normal body temperature.

The cost of the apparatus according to the invention is resolutely low, which makes it suitable for installation in any medical centre.

Certain components of the apparatus are disposable, thus ensuring aseptic conditions in the patient's heating stage.

The fixed parts of the apparatus are of simple reliable construction and housed in a lightweight portable casing that is easily handled.

## Claims

1. An apparatus for increasing the body temperature of a patient in a postoperative hypothermia status, comprising:
an air fluid or air/oxygen mixture fluid, delivering line (5) said line having an end associated with a fluid tank (7) and the other end provided with an inhaler, a hydrophobic filter (23), and heating means (20) for heating the fluid;
**characterized in that** it comprises
a heat exchanger (10) inserted on the line and licked by said fluid;
wherein the hydrophobic filter (23) is placed downstream the heat exchanger,
said heating means (20) being provided for heating the heat exchanger (10) in a controlled way.

2. An apparatus according to Claim 1, **characterized in that** said heating means comprise a heating nipper having jaws clamped onto said heat exchanger (10).

3. An apparatus according to Claim 1, **characterized in that** said hydrophobic filter (23) includes a water-proof but not-vapor-proof hydrophobic membrane (30).

4. An apparatus according to Claim 1, **characterized in that** said heat exchanger (10) and said filter (23) are incorporated in a portion (3) of the apparatus (1) associated with the patient, whereas said heating means (20) are incorporated in a portable unit (4) structurally independent from said portion (3).

5. An apparatus according to Claim 1, **characterized in that** the heat exchanger (10) comprises a pair of plates (15,16) separated from each other and stiffened by a set of foils (12) arranged to define winding or labyrinth-like paths for the fluid meant to pass through the heat exchanger.

6. An apparatus according to Claim 2, **characterised in that** said nipper (20) includes a thermal probe.

7. An apparatus according to Claim 4, **characterized in that** a power supply battery is housed within said portable unit (4).

8. An apparatus according to claim 2, **characterized in that** it comprises a water supply line (26) associated to the heat exchanger (10) for feeding water.

9. An apparatus according to claim 2, **characterized in that** the hydrophobic filter (23) is adapted to hold back condensation water due to the patient's breathing out.

## Patentansprüche

1. Vorrichtung zur Erhöhung der Köropertemperatur eines Patienten in einem postoperativen Zustand der Hypothermie, umfassend:
eine Zufuhrleitung (5) für ein Luftfluid oder ein Luft/Sauerstoff-Mischfluid, wobei die Leitung ein einem Fluidbehälter (7) zugeordnetes Ende hat und das andere Ende mit einem Inhalator versehen ist, einen hydrophoben Filter (23), und Heizeinrichtungen (20) zum Erwärmen des Fluids;
**dadurch gekennzeichnet, dass** sie
einen Wärmetauscher (10) umfasst, der in die Leitung eingesetzt ist und von dem Fluid überstrichen wird;
wobei der hydrophobe Filter (23) stromabwärts des Wärmetauschers angeordnet ist,
wobei die Heizeinrichtungen (20) vorgesehen sind, um den Wärmetauscher (10) gesteuert zu erwärmen.

2. Vorrichtung nach Anspruch 1, **dadurch gekennzeichnet, dass** die Heizeinrichtungen eine Wärmezange mit Backen umfassen, die auf den Wärmetauscher (10) geklemmt sind.

3. Vorrichtung nach Anspruch 1, **dadurch gekennzeichnet, dass** der hydrophobe Filter (23) eine wasserdichte, aber nicht dampfdichte hydrophobe Membran (30) umfasst.

4. Vorrichtung nach Anspruch 1, **dadurch gekennzeichnet, dass** der Wärmetauscher (10) und der Filter (23) in einem Abschnitt (3) der Vorrichtung (1) enthalten sind, der dem Patienten zugeordnet ist, während die Heizeinrichtungen (20) in einer vom Abschnitt (3) baulich unabhängigen, tragbaren Einheit (4) enthalten sind.

5. Vorrichtung nach Anspruch 1, **dadurch gekennzeichnet, dass** der Wärmetauscher (10) ein Paar voneinander getrennte Platten (15, 16) aufweist, die durch eine Gruppe von Flachkörpern (12) versteift sind, welche angeordnet sind, um sich windende oder labyrinthartige Wege für das Fluid zu bilden, das den Wärmetauscher durchströmen soll.

6. Vorrichtung nach Anspruch 2, **dadurch gekennzeichnet, dass** die Zange (20) einen Wärmefühler umfasst.

7. Vorrichtung nach Anspruch 4, **dadurch gekennzeichnet, dass** in der tragbaren Einheit (4) eine Stromversorgungsbatterie aufgenommen ist.

8. Vorrichtung nach Anspruch 2, **dadurch gekennzeichnet, dass** sie eine dem Wärmetauscher (10) zugeordnete Wasserzufuhrleitung (26) zum Zuleiten von Wasser aufweist.

9. Vorrichtung nach Anspruch 2, **dadurch gekennzeichnet, dass** der hydrophobe Filter (23) dazu ausgelegt ist, Kondensationswasser zurückzuhalten, das von der Ausatmung des Patienten herrührt.

## Revendications

1. Appareil pour augmenter la température corporelle d'un patient en état d'hypothermie post-opératoire, comprenant une conduite (5) d'acheminement d'un fluide de type air ou d'un fluide de type mélange air/oxygène, ladite conduite comprend une extrémité associée à un réservoir de fluide (7) tandis que l'autre extrémité comporte un inhalateur, un filtre hydrophobe (23) et des moyens de chauffage (20) pour chauffer le fluide ;
**caractérisé en ce qu'**il comprend un échangeur de chaleur (10) inséré sur la conduite et que vient lécher ledit fluide ;
et dans lequel le filtre hydrophobe (23) est disposé en aval de l'échangeur de chaleur, tandis que lesdits moyens de chauffage (20) servent à chauffer l'échangeur de chaleur (10) de manière contrôlée.

2. Appareil selon la revendication 1, **caractérisé en ce que** lesdits moyens de chauffage comprennent une pince de chauffage comportant des mâchoires serrées sur ledit échangeur de chaleur (10).

3. Appareil selon la revendication 1, **caractérisé en ce que** ledit filtre hydrophobe (23) comprend une membrane hydrophobe (30) imperméable à l'eau mais pas imperméable aux vapeurs.

4. Appareil selon la revendication 1, **caractérisé en ce que** ledit échangeur de chaleur (10) et ledit filtre (23) sont incorporés dans une partie (3) de l'appareil (1) associée au patient, tandis que lesdits moyens de chauffage (20) sont incorporés dans une unité portable (4) structurellement indépendante de ladite partie (3).

5. Appareil selon la revendication 1, **caractérisé en ce que** ledit échangeur de chaleur (10) comprend une paire de plaques (15, 16) séparées l'une de l'autre et raidies par un ensemble de feuilles métalliques (12) disposées de manière à définir des trajets de type enroulements ou labyrinthe pour le fluide devant passer à travers l'échangeur de chaleur.

6. Appareil selon la revendication 2, **caractérisé en ce que** ladite pince (20) comprend une sonde thermique.

7. Appareil selon la revendication 4, **caractérisé en ce qu'**une batterie d'alimentation électrique est logée dans ladite unité portable (4).

8. Appareil selon la revendication 2, **caractérisé en ce qu'**il comprend une conduite d'alimentation en eau (26) associée à l'échangeur de chaleur (10) pour acheminer de l'eau.

9. Appareil selon la revendication 2, **caractérisé en ce que** le filtre hydrophobe (23) est conçu pour retenir l'eau de condensation générée lors de l'expiration du patient.
